# EUROPEAN PATENT SPECIFICATION

(11) **EP 1 447 064 B1**
(45) Date of publication and mention of the grant of the patent: **02.06.2010**
(21) Application number: 04003200.5
(22) Date of filing: 12.02.2004
(51) Int. Cl.: A61F 9/011, A61B 17/28, A61B 17/30

(54) **Kit-of-parts for surgically ablating eye tissue**
Elementensatz zur chirurgischen Ablation von Augengewebe
Ensemble d'éléments pour l'ablation chirurgicale de tissu des yeux

(30) Priority: 12.02.2003 US 446773 P
(43) Date of publication of application: 18.08.2004
(73) Proprietor: Coherent GmbH, 37079 Göttingen (DE)
(72) Inventor: Holz, Frank G., D-53127 Bonn (DE); Bindewald, Almut, D-53129 Bonn (DE); Specht, Holger, D-73434 Aalen Unterrombach (DE); Schütt, Florian, D-69120 Heidelberg (DE)
(74) Representative: HOFFMANN EITLE

(56) References cited:
- WO-A-91/06271
- DE-A- 19 852 574
- US-A- 5 738 676
- US-A1- 2002 111 608

## Description

### Background of the invention

This invention relates to a kit-of-parts that is particularly useful for the surgical removal of choroidal neovascularizations which are associated with macular degeneration.

Age-related macular degeneration (AMD) is the leading cause of legal blindness in the western nations beyond 50 years of age. The most frequent cause for severe visual loss associated with AMD is the growth of neovascular membranes from the choroid into the subretinal space [Freund, K.B., Yannuzzi, L.A., J.A. Sorenson: Age-related macular degeneration and choroidal neovascularization, Am. J. Ophthalmol. 115 (1993) 786-791]. This usually results in irreversible degeneration of the overlying retina. Various therapeutic modalities have been brought forward in patients with AMD including thermal laser photocoagulation [Macular Photocoagulation Study Group: Argon laser photocoagulation for neovascular maculopathy: three-year results from randomized clinical trials, Arch. Ophthalmol. 104 (1986) 694-701], surgical removal of the neovascular net with or without transplantation of retinal pigment epithelial or iris pigment epithelial cells [Algvere, P.V., Beglin, L., Gouras, P.Y. Sheng: Transplantation of fetal retinal pigment epithelium in age-related macular degeneration with subfoveal neovascularization, Graefes Arch. Clin. Exp. Ophthalmol. 232 (1994) 707-716], macular translocation/rotation [Eckardt C., Eckardt U., H.G. Conrad: Macular rotation with and without counter-rotation of the globe in patients with age-related macular degeneration, Graefes Arch. Clin. Exp. Ophthalmol. 237 (1999) 313-325], photodynamic therapy [Woodburn, K.W., Engelman, C.J., M.S. Blumenkranz: Photodynamic therapy for choroidal neovascularization: a review, Retina 22 (2002) 527-528] or pharmacological therapy using antiangiogenic drugs [Challa, J.K., Gillies, M.C., P.L. Penfold: Exsudative degeneration and intravitreal triamcinolone, 18 month follow up, Aust. N.Z.J. Ophthalmol, 26 (1998) 277-281], and radiotherapy [RAD Study Group: A randomized, prospective, double-blind clinical trial on radiation therapy for subfoveal choroidal neovascularization secondary to age-related macular degeneration, Ophthalmology 106 (1999) 2239-2247]. However, the visual prognosis is still dismal for most of the patients. As age-related macular degeneration represents a major health burden with increasing prevalence due to demographic changes development of novel and effective modes of therapy appears mandatory.

Microsurgical excision of neovascular membranes via a small retinotomy temporal superior to the macula during pars plana vitrectomy is technically relatively easily done but is usually associated with inadvertent removal of the underlying RPE due to tight adherence of the RPE to the membrane. As functional RPE cells are a prerequisite for normal photoreceptor function, this usually leads to a blind spot in the central retina and, therefore, to loss of reading ability etc. Only an intact, functioning monolayer of RPE cells beneath the neurosensory retina could maintain regular metabolism and retinal function [Bird, A.C.: Age-related macular disease, Br. J. Ophthalmol. 80 (1996) 1-2; Holz, F.G., D. Pauleikhoff, R.F. Spaide, A.C. Bird: Age-related Macular Degeneration, Springer-Verlag (2003)].

Several attempts have been undertaken to replace removed RPE during surgical excision of subfoveal choroidal neovascularizations. Allogenic RPE was apparently rejected and resulted in accumulation of extracellular fluid with chronic macular edema and loss of visual function [Rezai, K.A., Semnani, R.T., Farrokh-Siar, L., Hamann, K.J., Patel, S.C., Ernest, J.T., G.A. van Seventer: Human fetal retinal pigment epithelial cells induce apoptosis in allogenic T-cells in a Fas ligand and PGE2 independent pathway, Curr. Eye Res. 18, (1999) 430-439]. Autologous cultured iris pigment epithelial cells injected subretinally as cell suspension were not associated with functional recovery [Thumann, G., Aisenbrey, S., Schraermeyer, U., Lafaut, B., Esser, P., Walter, P., K.U. Bartz-Schmidt: Transplantation of autologous iris pigment epithelium after removal of choroidal neovascular membranes, Arch. Ophthalmol. 118 (2000) 1350-1355]. Another approach has been the excision from extramacular RPE/choroid complexes that were transferred at the subretinal site where the neovascular tissue had been removed before [Stanga, P.E., Kychenthal, A., Fitzke, F.W., Halfyard, A.S., Chan, R., Bird, A.C., G.W. Aylward: Retinal pigment epithelium translocation and central visual function in age-related macular degeneration: preliminary results, Int. Ophthalmol. 23 (2001) 297-307; Stanga, P.E., Kychenthal, A., Fitzke, F.W., Halfyard, A.S., Chan, R., Bird, A.C., G.W. Aylward: Retinal pigment epithelium translocation after choroidal neovascular membrane removal in age-related macular degeneration, Int. Ophthalmology 109 (2002), 1492-1498; van Meurs, J., Averst, E., Stalman, P., Kuijpers, R., Hofland, L., Baarsma, S., M. van Hagen: The translocation of autologous retinal pigment epithelial cells in patients with subfoveal choroidal neovascularization, Club Gonin Meeting 2002; J.C. van Meurs and P.R. van den Biesen: Autologous Retinal Pigment Epithelium and Choroid Translocation in Patients With Exsudative Age-related Macular Degeneration: Short-term Follow-up, Am J Ophthalmol 136 (2003) 688-695]. Although retinal function was detected over these grafts, visual outcome was overall unsatisfactory.

DE 198 52 574 A1 pertains to a surgical instrument for phacoemulsification by destroying and aspirating the lens of an eye. The instrument for destroying the lens is provided in D1 by a laser emitting high energy radiation which step-by-step ablates the lens and aspirates the parts produced during ablation.

US 5,738,676 pertains to a laser surgical probe for use in intraocular surgery, and is considered to represent the closest prior art. This probe includes means to hold tissue and means to excise it, in the vicinity of the retina.

### Summary of the invention

It is an object of the present invention to provide means for more effectively treating tissue, particularly damaged or degenerated tissue, e.g. choroidal neovascularizations.

It is another object of the present invention to provide means for more effectively treating macular degeneration.

These and other objects are solved by a kit-of-parts as defined in claim 1 below. Further advantageous embodiments are recited in the dependent claims.

The invention permits to excise a sheet of tissue from surrounding tissue, treat the isolated sheet with a laser light thereby ablating at least one layer of said sheet, or a part thereof, and to insert the thus-treated sheet to the location from which said sheet of tissue was excised or to another location.

It actually is not required to ablate the entire choroidal tissue, rather it is possible to ablate or emulsify just the choroidal tissue surrounding the vessels. The vessels, and particularly the arteries, are less easily ablated and are mechanically relatively strong. After such an ablation or emulsification, they can easily be removed mechanically by the surgeon by just pulling them from the remaining sheet to which they have little adhesion after the remaining choroidal tissue is gone.

The treated sheet can preferably be translocated to a location from which damaged or degenerated tissue has been excised. The damaged or degenerated tissue are preferably choroidal neovascularizations from the neurosensory retina. The sheet of non-damaged or non-degenerated tissue is preferably one comprising choroidal tissue, Bruch's membrane and retinal pigment epithelial cells. In this case, the laser light treatment results in an ablation of the bulk of choroidal tissue. The invention is useful for the treatment of neovascular macular degeneration.

### Brief description of the drawings

- Fig. 1: is a graphical illustration of a method to be carried out with the kit of the present invention, for the treatment of macular degeneration;
- Fig. 2: is a photograph showing the front and side view of ablating means, embodied as a laser tip with a modified aperture (300 µm) that was used in the experiments;
- Fig. 3: is a cross-sectional view of one embodiment of the inventive means for excising and ablating tissue;
- Fig. 4a: is a perspective view of a first embodiment of the inventive means for holding excised tissue;
- Fig. 4b: is a perspective view of a second embodiment of the inventive means for holding excised tissue;
- Fig. 5: is a side view of an embodiment of the inventive means for maintaining the distance between the means for excising and ablating tissue on the one hand and the means for holding excised tissue on the other hand; and
- Fig. 6: is a perspective view of an embodiment of the inventive kit-of-parts in operation.

### Detailed description of the preferred embodiments

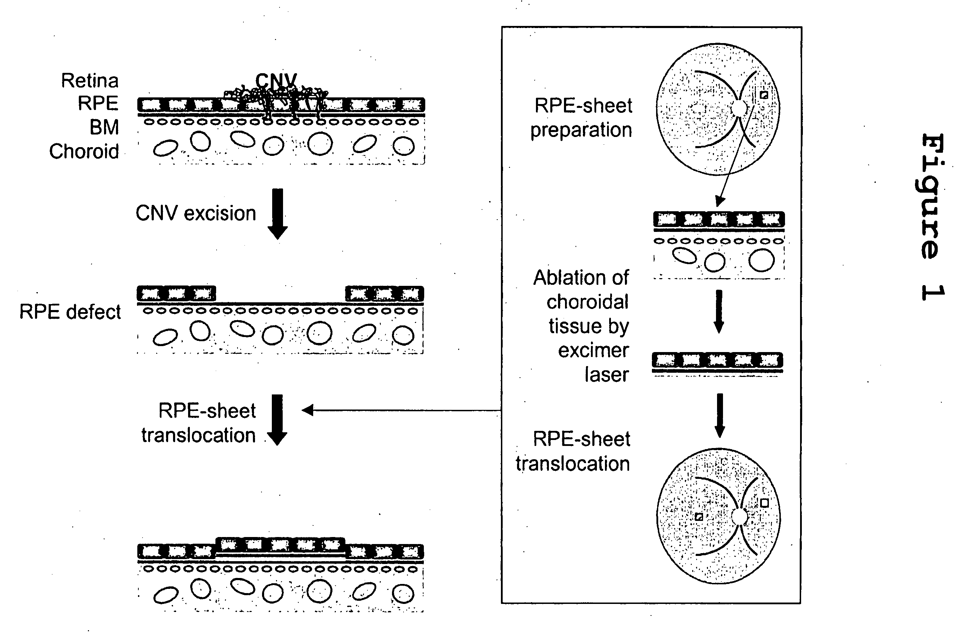

The present invention permits to isolate a sheet of tissue from surrounding tissue and then treat it with laser light to ablate at least one layer of said sheet, or a part thereof (Fig. 1).

The isolated tissue may be damaged or degenerated. Preferably, however, it is a non-damaged or non-degenerated tissue isolated from another region of the patient's body (e.g. patient's eye). The terms "non-damaged" and "non-degenerated" mean that no physiological dysfunction of the tissue is apparent.

The tissue is composed of two or more layers. At least two layers of this laminate have different ablation characteristics at the wavelength used for ablation. The term "different ablation characteristics" means that the layers show different ablation thresholds at the laser wavelength. The difference in the ablation characteristics are generally inherent to the tissue. However, in those cases where the inherent differences are not sufficient for a proper ablation, light absorbers (e.g. UV absorbing substances) may be subjected specifically to the layer to be ablated. The ablation characteristics can be determined by means of a empirical determination of the necessary ablation threshold for each layer. Investigations have shown that the choroidal capillaris serve as boundary layer with restraining function to the ablation with 308 nm laser light.

The surface area of one side of the isolated sheet is preferably in the range of 1 to 30 mm², more preferably 5 to 20 mm². In the case that the isolated sheet is to be transplanted after the laser treatment, the size of the isolated sheet is preferably selected such that it corresponds to the size of the excised damaged or degenerated tissue. The tissue preferably includes choroidal tissue, Bruch's membrane and RPE cells and is preferably derived from the peripheral retina. The isolation can be carried out by surgical excision. Initially, this site is demarcated with laser to avoid bleeding from retinal or choroidal vessels. Excision is then performed with retinal scissors. This step could potentially also be carried out with an Excimer laser. The autologous patch to be translocated is then held with forceps.

The isolated sheet is subsequently treated with a laser light in a manner that allows the ablation of that tissue layer having the lower ablation threshold at the laser light wavelength. This can be achieved by selecting a laser that operates at a wavelength at which the tissue layer to be ablated has a lower ablation threshold than the adjacent tissue layers. The laser light wavelength can be in the UV/VIS range (i.e. from 180 to 800 nm).

For the ablation of choroidal tissue, the laser is one emitting light at a wavelength in the range of 180 to 400 nm. For example, a laser operating at 193 nm (e.g. a ArF Excimer laser) may be used. However, longer wavelengths in the range of 280 to 400 nm are preferred, a laser operating at 308 nm (e.g. a XeCl Excimer laser) being particularly preferred. Alternatively, laser diodes, femtosecond lasers, Er:YAG lasers or Nd:YAG lasers, which may be frequency-multiplied, may be used. Longer wavelengths allow the ablation to be carried out in the eye, i.e. intraocularly.

The 308 UV Excimer laser is known to be suitable for the ablation of human tissue including skin cartilage [Kaufmann, R., R. Hibst: Pulsed Er:YAG- and 308 nm UV-Excimer laser: an in vitro and in vivo study of skin ablative effects, Lasers Surg. Med., 9 (1989) 132-140; Prodoehl, J.A., Rhodes, A.L., Commings, R.S., Meller, M., H.H. Sherk: 308 nm Excimer laser ablation of cartilage, Lasers Surg. Med. 15 (1994) 263-268]. Ocular surgery applications include cataract surgery [Martinez, M., Maguen, E., Bardenstein, D., Duffy, M., Yoser, S., Papaioannou, T., W. Grundfest: A comparison of Excimer laser (308 nm) ablation of the human lens nucleus in air and saline with a fiber optic delivery system, Refract. Corneal Surg. 8 (1992) 368-374] and laser ablation of trabecular meshwork for glaucoma treatment [Vogel, M., K. Lauritzen: Selective Excimer laser ablation of the trabecular meshwork, Clinical results, Ophthalmologe 94 (1997) 665-667 and Walker. R, Specht. H:Theoretical and physical aspects of Excimer laser trabeculotomy (ELT) ab interno with the AIDA laser with a wave length of 308 mm, Biomed Tech 2002 May; 47 (5) : 106-10].

The laser is preferably a pulsed laser. The pulse duration is not restricted and may be as short as 1 fs, for example in the range of 1 fs to 100 ps. The preferred pulse durations are within the range of 10 to 300 ns, more preferably 30 to 150 ns, most preferably 60 to 80 ns. For 308 nm the pulse energy at the tissue to be ablated is preferably in the order of 3 to 300 mJ/mm², more preferably 25 to 50 mJ/mm², most preferably 35 to 40 mJ/mm².

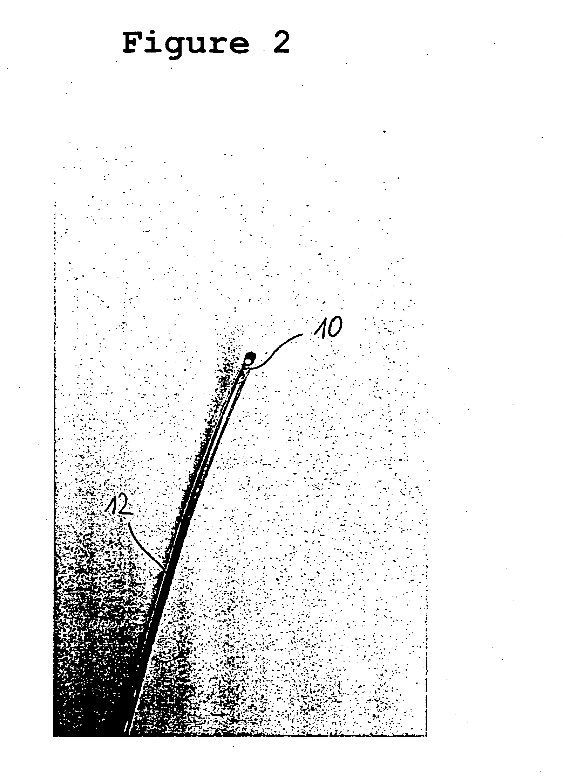

The laser radiation is transported to the application site by means of light guides, e.g. optical fibers such as quartz fibers, or hollow light guides. In a preferred embodiment, the light guide is a fiber optic delivery system integrated in a laser tip. Fig. 2 shows an example for such a laser tip. It has been given reference sign 10. The optical fiber carries reference sign 12. The diameter of the optical fibers 12 is preferably 50 to 2000 µm, more preferably 100 to 800 µm, most preferably about 200 to 600 µm. The distal exit area of the light guide is preferably in the range of 0.002 to 3 mm², preferably 0.008 to 0.3 mm², most preferably about 0.03 mm².

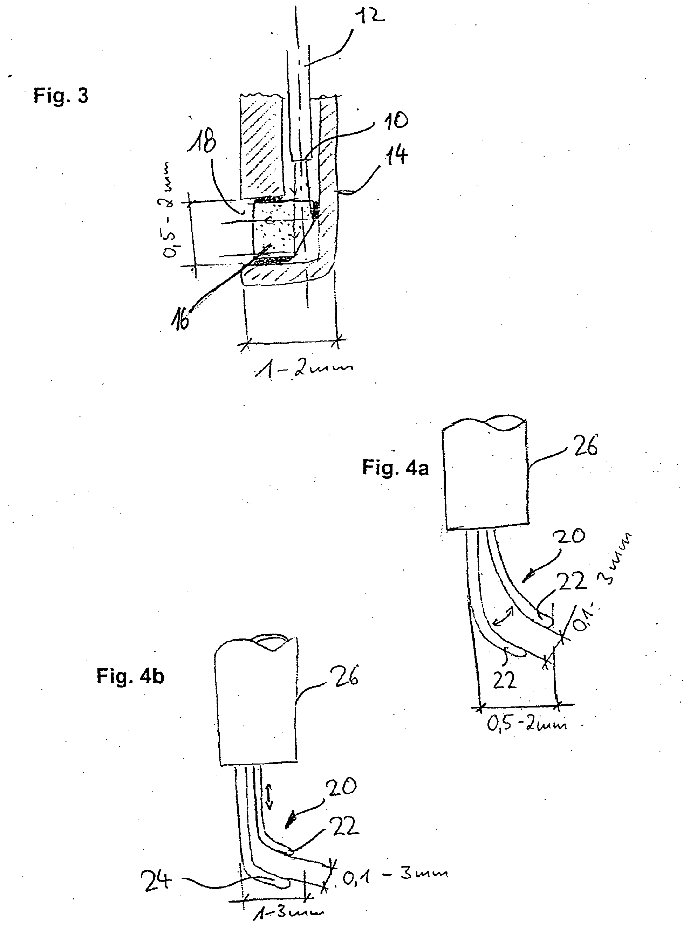

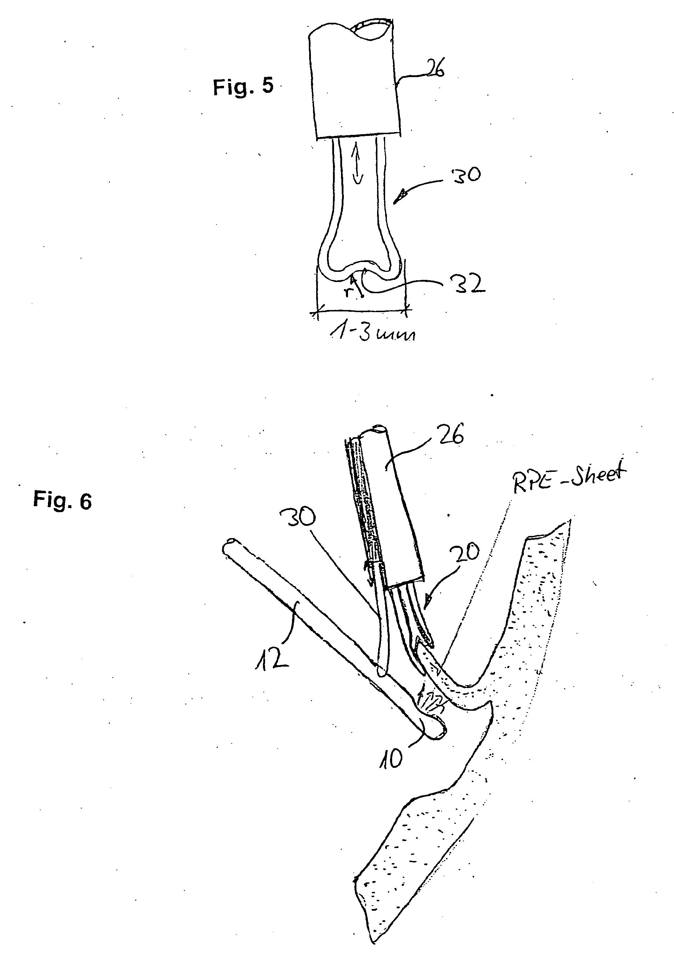

For the treatment the isolated sheet is held with a holding means such as forceps (Figs. 4a, 4b) which leave a large portion of the tissue untouched rather than being laid against a surface, especially in the case of epithelium cells which are easily irreversibly damaged by mechanical forces. The tissue therefore is preferably held suspended in gas or liquid, like body fluids or sterile fluids. The tissue sheet can held suspended freely for treatment or preferably held at one end while another end remains attached to its former location in the patient's body and cut loose only after treatment. The surgeon can hold this distance and angle manually under direct visual control or more preferably visual control aided by a microscope and / or magnification lenses or an endoscope. The surgeon can be aided by mechanical (Fig. 5) or magnetic guidance systems connected to said means holding the isolated sheet. Additionally, or alternatively the surgeon can be aided by control systems measuring the distance and / or angle and providing the surgeon with visual, audible or tactile feedback. For example the instrument can be mechanically connected to the instrument allowing only movement in dimensions parallel to the sheet to be treated. Movement in this direction is preferably limited to a substantially axial movement of between about 0.5 and 4 mm. The surgeon's flexibility is greatly enhanced if the fiber can also rotate about the longitudinal axis. It is preferred if the fiber can rotate through an angle of approximately 120°. However, satisfactory results can already be achieved if the angle is limited to approximately 5°, more preferably approximately 10°, still more preferably approximately 15°. The surgeon can also be assisted by robotic surgical tools.

The surgical instrument guiding the light guide consists in a preferred embodiment of an optical fiber or bundle of optical fibers surrounded in a cannula of rigid material like stainless steel or passing through the working channel, or lumen, of an endoscope. The instrument is shaped to facilitate the introduction into the patient's body to the site of the treatment of the tissue sheet. In retinal surgery this preferably means a straight instrument. The tip of the instrument where the laser light exits the light guide is shaped to allow close approximation of the light quide to said tissue sheet while avoiding contact of the instrument with said tissue sheet and minimizing the damage in case of accidental contact. This can be achieved by a bevelled tip of the instrument and / or light guide, where the sum of the bevel of the tip and the angle at which the axis of the instrument is positioned relative to the tissue sheet defines the angle between the laser tip and the surface of the tissue sheet. To avoid situations resulting in counter-intuitive beam paths after the light exits the light guide, the tip of the instrument can be equipped with a reflective surface or a lens.

When the instrument is to be used in air, the angle of the bevel may be between 90° and about 50°. The laser beam would then exit at an angle of between 0° and about 35° relative to the longitudinal axis of the fiber. When the instrument is to be used in water, the angle of the bevel may be between 90° and about 20°. The laser beam would then exit at an angle of between 0° and about 28° relative to the longitudinal axis of the fiber.

At a bevel of about 60° the instrument could be tested in air (at an exit angle of about 18.5°), and would function in most liquids with an index of refraction similar to that of water (at an exit angle of about 5°), so that this constitutes one preferred embodiment. Alternatively, the bevel can be between about 40° and 50°at which the beam in air would be deflected by about 90° (Sidefire - Air), or the bevel can be between about 20° and 40°at which the beam in water would be deflected by about 90° (Sidefire - Water). 90° deflection is preferred by some practitioners in terms of testing or handling.

Fig. 3 shows a possible embodiment, where the tip 10 of the optical fiber 12 is equipped with a cap 14. The cap can be made from quartz glass, or from stainless steel. Cap 14 advantageously further ensures that any laser beams do not impinge upon tissue that is not to be treated with laser radiation. To this end, cap 14 in the preferred embodiment encloses tip 10 of optical fiber 12 entirely, but for a lateral exit window 18.

Instead of having the optical fiber 12 be equipped with a bevelled tip, a prism 16 can also be used. The prism 16 serves to deviate the laser beam exiting the tip 10 of the optical fiber 12, and if a prism 16 is used, tip 10 may have an exit surface substantially perpendicular to the longitudinal axis of optical fiber 12. These prisms per se are known in the art, so that a detailed description is dispensed with.

If a bare fiber is used with a bevelled tip, polished at an angle significantly different from 90°, the instrument is preferably operated in a liquid with an index of refraction not much lower, about equal or even higher than that of the light guide (in glass and quartz fibers the index of refraction is typically between about 1.2 and 1.6 for the wavelengths transmitted, while pure water has an index of refraction of about 1.2 to 1.5, depending on the wavelength) and having a low absorption coefficient at the laser light wavelength. Suitable liquids include physiological saline. Also, a bundle of fibers of small diameter can be used. While the individual fibers have perpendicular exit surfaces, the fibers are arranged with some fibers protruding further than others, so that their exit surfaces approximate a bevelled tip. Such a bundle preferably is packed densly (hexagonal) but can be formed to the desired shape, with circular shapes being easy to produce and rectangular or linear shapes being particularly useful for precisely defined treatment of particular areas of the sheet.

The instrument surrounding the lightguide (cannula or endoscope) is preferably shaped with rounded edges to allow closer approximation and to avoid damage in case of accidental contact with the tissue sheet to be treated (not shows). The lightguide exit surface can end to form an approximately smooth surface with the tip of the instrument or protrude (especially preferable in the case of an endoscope) or be withdrawn.

Similarly, forceps 20 (Figs. 4a, 4b) and distance holding means 30 can be embodied as an endoscope. Preferably, both form part of the same endoscope.

Forceps 20, for instance, can be made from a shape-memory metal, for instance a nickel titanium alloy, preferably Nitinol®. Having the shape-memory metal be programmed into the form of grippers 22, 24 permits that the forceps 20 spring into a gripper shape, upon withdrawal of a sheath 26. Of course, alternatively sheath 26 can be held stationary and forceps 20 be advanced relative to the sheath 26. The shape of the grippers 20, 24 can be rounded (Fig. 4a) or angular (Fig. 4b). It is advantageous if the distal ends of the grippers project laterally from the longitudinal axis defined by the lumen along which forceps 20 is guided within sheath 26. If the shape of the grippers is rounded, and both grippers are permitted to move (Fig. 4a) upon axial displacement of the sheath 26, a lateral projection by between about 0.5 and 2 mm has been found to be advantageous. A spacing between the gripping surfaces of grippers 22 of between about 0.1 to 3 mm has proven to be effective. For a rectangular shape (Fig. 4b) and one movable gripper 22 and one stationary gripper 24, a lateral projection of between about 1 and 3 mm is preferred. In this embodiment the gripping surfaces of grippers 22, 24 are also spaced apart by between about 0.1 and 3 mm.

Distance holding means 30 is formed into a loop shape, as may be taken from Fig. 5. The distal end region of distance holding means 30 exhibits a convex radius 32. The radius curvature R of radius 32 is advantageously between about 1 and 2 mm. This will ensure that the shape of the distal end region of distance holding means 30 conforms to the shape of optical fiber, or fiber bundle 12 (Fig. 6).

Fig. 6 exemplifies that distance holding means 30 as well as forceps 20 can be embodied in a single endoscopic structure having a multi-lumen sheath 26.

When ablating choroidal tissue, the laser operating parameters such as the energy output are selected such that the choroidal tissue is completely ablated and the Bruch's membrane essentially remains intact. At any rate, the laser ablation treatment is discontinued before the RPE layer is ablated. To ensure this, the laser ablation is preferably controlled by monitoring one or more optical properties or one or more mechanical properties of the sheet of tissue or the ablation plume. When the monitored property changes significantly, the laser ablation is stopped. Alternatively, the energy level of the next laser pulse is adjusted accordingly. An audible, visual or tactile signal may be provided to inform the surgeon of this change. For example, the fluorescence and/or the reflection of the sheet of tissue can be monitored, said fluorescence or reflection being induced by the laser used for ablation, including a pilot beam or pulse thereof, or by a second laser. The monitored mechanical property can be the sound or pressure waves emitted by the ablation.

During laser ablation, an ultraviolet absorbing compound may be administered to the patient to protect the eye from scattered laser light. Moreover, a compound that is useful for the therapeutic treatment of macular generation may also be administered. The ultraviolet absorbing and/or therapeutically active compound is preferably administered intraocularly. These compounds include glutathione enhancing agents as disclosed in US 5,596,011, such as N-acetyl cysteine; vitamins such as ascorbic acid, alpha-tocopherol (vitamin E), beta-carotone, retinal (vitamin A), lutein, zeaxanthin; and inhibitors of the protein tyrosine kinase pathway such as genistein.

After the laser ablation, the thus-treated sheet is inserted to the location from which said sheet of tissue was excised or to another location. It is particularly preferred to translocate the thus-treated sheet to another location from which another tissue has been excised, preferably damaged or degenerated tissue. In such a case, the present invention can be used in a transplantation method, i.e. the sheet of tissue is translocated or transplanted to the location from which the damaged or degenerated tissue (e.g. the choroidal neovascularizations or neovascular membranes) was excised. In the case of treating choroidal neovascularizations, e.g. those associated with age-related macular degeneration, this is preferably done in a manner such that the Bruch's membrane of the treated sheet of tissue is in contact with the Bruch's membrane of the location from which the choroidal neovascularizations were excised. Hence, the present invention allows the translocation of intact autologous RPE sheets.

After these steps the essential part of the procedure is done and the surgery is completed with a temporary internal tamponade which is either silicon oil or air and C₃F₈ gas (C₃F₈ or SF₆). These tamponades are commonly used in vitreoretinal surgery.

In the first step of the transplantation method for which the present invention is suitable, damaged or degenerated tissue is excised. The damaged or degenerated tissue is preferably from the eye and preferably comprises (subfoveal) choroidal neovascularization (CNV). It is preferably excised from the neurosensory retina, most preferably from the macular region (macula lutea) that may be associated with late-stage age-related macular degeneration.

The choroidal neovascularizations (CNV) may be identified and subsequently removed as follows: Sequalae of choroidal neovascularizations such as hemorrhage within and/or under the retina as well as swelling of the retina (macular edema) can be seen by funduscopy. The neovascular membranes are identified with the help of fluorescein and indocyanine green angiography. The neovascular net is then removed by performing a small retinotomy temporal to the macula. Subsequently balanced salt solution is injected into the subretinal space to separate the neurosensory retina from the RPE and the neovascular membrane at the posterior pole. Subretinal forceps are then introduced into the subretinal space and the CNV is removed via the retinotomy site and, subsequently, through a sclerotomy. The surgical excision of CNV is usually associated with the removal of corresponding retinal pigment epithelium (RPE), so that photoreceptors will be in direct contact with Bruch's membrane. This will inevitably lead to irreversible degeneration and cell death of the neuronal outer retinal cellular elements.

This invention is particularly useful for the treatment of macular degeneration, histoplasmosis, pathological myopia, angioid streaks, anterior ischemic optic neuropathy, bacterial endocarditis, Best's disease, birdshot retinochoroidopathy, choroidal hemangioma, choroidal nevi, choroidal nonperfusion, choroidal osteomas, choroidal rupture, choroideremia, chronic retinal detachment, coloboma or the retina, Drusen, endogenous Candida endophthalmitis, extra-papillary hamartomas of the retinal pigmented epithelium, fundus flavimaculatus, idiopathic (sic), macular hold, malignant melanoma, membrane proliferative glomerulonephritis (type II), metallic intraocular foreign body, morning glory disc syndrome, multiple evanescent white-dot syndrome, neovascularization at ora serrata, operating microscope burn, optic nerve head pits, photocoagulation, punctate inner choroidopathy, radiation retinopathy, retinal cryoinjury, retinitis pigmentosa, retinochoroidal coloboma, rubella, sarcoidosis, serpoiginous or geography choroiditis, subretinal fluid drainage, titled disc syndrome, Taxoplasma retinochoroiditis, tuberculosis or Vogt-Koyanagi-Harada syndrome.

### Example

### Method:

Freshly enucleated human donor eyes that were obtained for corneal transplantation were used. The anterior portion including the cornea, the lens, the iris and anterior sclera was excised and the remaining open eye cup placed on an eye cup holder under a photomicroscope (Wild, Herbrugg, Switzerland). The eye was filled with balanced saline solution (BSS) and illuminated by a flexible halogen fiber optic (Schott, Germany). A fiber optic delivery system integrated in a laser tip and a 308 nm AIDA UV-Excimer laser (Glautec AG, Switzerland) were used. The laser tip showed an angle of approx. 45° (Fig. 2). The rectangular grinded laser tip aperture was 7 x 30 µm². Laser energy was modified using an infinitely variable aperture. Energy ranged from 0.19 to 0.38 µJ. Pulse length was 60 ns. The retina was removed by small forceps and a 3 x 3 mm² graft of RPE, Bruch's membrane and choroid was excised. The graft was held with small forceps and the laser tip forwarded towards the choroidal side in a distance between 1 to 3 mm. Photographs of ablated choroid were taken using the photomicroscope and a camera (Olympus BX 50, Hamburg, Germany). Ablated RPE/choroid tissue was fixed in formaldehyde, dehydrated by ethanol, stained with hematoxilin-eosin and embedded in paraffin for light microscopy. For electron microscopy the tissue was fixed for 2 hours with 3% glutaraldehyde in 0.1 M cacodylate buffer, post fixed for 2 hours in 1% osmium tetroxide in 0.1 M cacodylate buffer before being dehydrated in ethanol and propylenoxide. Afterwards the tissue was embedded in Epon and Araldit (Serva) for 3 days. Sections were cut on a Reichert-Jung ultramicrotome and mounted on copper grids (Plano, Wetzlar, Germany) before staining with uranyl and lead citrate. Electron microscopy was performed using an Elo-TSD, (Zeiss, Germany).

### Results:

During the laser treatment, laser pulses at the tip created a dim blue flash and depending on the energy applied small gas bubbles were noted. A broad spectrum of laser energy (0.5 mJ to 1.0 mJ for the 200 µm laser fibre, 1.2 to 2.1 mJ for the 600 µm laser fibre) was necessary to ablate effectively due to large interindividual differences in tissue diameters and properties. Accidental contacts of the laser tip did not damage the layer and caused no perforation. Treated specimen were cut by a scalpel and fixed as described in the methods section. Light microscopic examination showed cleanly and accurately separated tissue borders without thermal collateral damage. Measurements of choroidal thickness showed a reduction of up to 80% compared with untreated specimen from the same eye. Variable amounts of inner choroidal tissue was still present at the basal side of Bruch's membrane. Morphologically the overlying RPE cells appeared normal. Electron microscopy confirmed the result of light microscopy.

### Discussion:

In this experimental study we demonstrated that choroidal tissue can be effectively microablated from a RPE/Bruch's membrane/choroid graft with a 308 nm UV-Excimer laser. A probe was designed and used in these experiments that could also be applied during surgery for intraocular ablation. Application of this technique for RPE-transplantation in eyes with neovascular age-related macular degeneration might yield better functional results when compared with grafts that have normal, unablated choroidal tissue. It has been demonstrated that it is possible to precisely ablate tissue without thermal or other collateral damage. This may relate to the small ratio between radiation-absorbing tissue to water. Noncontact laser-guided removal of tissue allows the application of laser effects without mechanical damage to the tissue.

Recently autologous RPE/Bruch's membrane choroid grafts have been translocated in eyes with prior excision of subfoveal neovascular membranes that led to markedly impaired central visual function before intervention. Hereby, the goal is to transplant functional RPE-cell monolayer sheets with minimal trauma under the neurosensory retina. Minimization of trauma and maintenance of an intact RPE monolayer appears crucial as RPE cells may otherwise dedifferentiate, and, consequently, loose their functional properties essential for normal photoreceptor function. Both subretinal injection of autologous RPE or iris pigment epithelial cells have not led to satisfactory results, as these cell suspension did not form monolayers, but showed clumping and apparent dedifferentiation [Binder, S., Stolba, U., Krebs, I., Kellner, L., Jahn, C., Feichtinger, H., Povelka, M., Frohner, U., Kruger, A., Hilgers, R.D., W. Krugluger: Transplantation of autologous retinal pigment epithelium in eyes with foveal neovascularization resulting from age-related macular degeneration: A pilot study, Am. J. Ophthalmol. 133 (2002) 215-225]. Therefore, the use of grafts with supporting extracellular matrix such as Bruch's membrane has a good rational. However, the choroid is firmly attached to Bruch's membrane or the overlying RPE cell layer. The use of the 308 nm Excimer laser allows the intraocular treatment, i.e. without bringing the graft out of the intraocular milieu in an adverse environment that might traumatize the RPE cells and cause later dysfunction when repositioned under the retina. The laser probe here could be introduced into the vitreous cavity via the small openings of normal 0.9 mm sclerotomies that are created regularly for vitreoretinal surgery. Thus, choroidal tissue from the graft could be ablated within the eye.

The AIDA UV-Excimer laser thus is a suitable tool for an atraumatic thinning of the choroid. The interaction between laser light and tissue depends on absorption coefficient of target tissue, time constants (e.g. exposure time) and delivered energy. The wavelength of 308 nm has an absorption coefficient of 0.01 cm⁻¹ in water. This equals a penetration of 1 meter. The absorption coefficient of tissue is much larger (50 cm⁻¹). The resulting optical penetration is less than 200 µm. These small ablation rates in the range of µm facilitate an atraumatic preparation of RPE/choroid complexes due to controlled removal of tissue. Furthermore, the AIDA UV-Excimer laser shows only 2 µm diffusion of thermal energy per pulse. Thermal damage is therefore limited to the boundaries of laser treatment leaving overlying RPE intact. Gas bubble generation is not caused by explosive water evaporation but related to slow heat transport from tissue to water.

In summary, the present invention may be used to ablate intraocularly prepared RPE/choroid transplants that might improve visual outcome of patients with neovascular age-related macular degeneration.

## Claims

1. Kit-of-parts for treating neovascular macular degeneration and more particularly choroidal neovascularization, the kit comprising:
- means (10, 12) for intraocularly excising retinal tissue,
- means (20) for intraocularly holding the excised retinal tissue, and
- means (10, 12) for intraocularly ablating the excised retinal tissue, wherein
- the means for intraocularly ablating the excised retinal tissue can guide and emit energy, preferably laser light, and more preferably laser light having a wave length in the range of 180 to 400 nm, further comprising
- means (30) for holding the distance between the means for intraocularly holding the excised retinal tissue and the means for intraocularly ablating the excised retinal tissue, wherein the shape of at least a distal portion of the means for intraocularly ablating the excised tissue and the shape (32) of the distal end of the means for holding the distance (30) are adapted so as to conform to one another, wherein the distal end of the means for holding the distance comprises a concave radius of curvature (32), preferably of between about 1 and 2 mm, wherein the
- means for intraocularly ablating the excised retinal tissue comprises at least one optical fiber (12) terminating in a prism (16) for deviating the laser light, and wherein the
- means for intraocularly holding the excised retinal tissue comprises an endoscopic forceps (20) having two grippers (22, 22; 22, 24), and further comprising
- an endoscope having a sheath (26), and at least one lumen within the sheath, wherein the means (20) for intraocularly holding the excised retinal tissue passes through a lumen of the endoscope, and the means for holding the distance (30) passes through a lumen of the endoscope, wherein the distance holding means (30) is movable upon actuation by movement relative to the sheath (26),
- further comprising means for generating laser light, the means for generating laser light being operable to generate laser light having a wavelength in the range of 180 to 400 nm, preferably a wavelength of 193 nm and/or 308 nm, wherein the means for generating laser light is an Excimer laser.

2. Kit-of-parts as claimed in claim 1, **characterized in that** the at least one optical fiber (12) has an index of refraction of about 1.2 to 1.5, and preferably is a quartz fiber, or a hollow light guide.

3. Kit-of-parts as claimed in claim 1 or 2, **characterized in that** the diameter of the at least one optical fiber (12) is from 50 to 2000 µm, preferably from 100 to 800 µm, more preferably from about 200 to 600 µm.

4. Kit-of-parts as claimed in any of claims 1 to 3, **characterized in that** the at least one optical fiber (12) has a distal light exit surface (10; 18) with a surface area in the range of 0.002 to 3 mm², preferably 0.008 to 0.3 mm², more preferably about 0.03 mm².

5. Kit-of-parts as claimed in any of claims 1 to 4 **characterized in that** the means for intraocularly ablating the excised retinal tissue comprises at least one optical fiber having mounted to its distal end a cap (14) with a light exit (18), preferably a lateral light exit.

6. Kit-of-parts as claimed in any of claims 1 to 5, **characterized in that** the means for intraocularly ablating the excised retinal tissue has a longitudinal axis, and can be displaced in the direction of the longitudinal axis by between about 0.5 and 4 mm, and/or can be rotated about the longitudinal axis through about 5°, preferably through about 10°, more preferably through about 15°, still more preferably through about 120°.

7. Kit-of-parts as claimed in any of claims 1 to 6, wherein the means for generating laser light is operable to generate pulsed laser light, the pulses having a duration of between about 10 ns to 300 ns, and/or an energy in the range of about 3 to 300 mJ/mm²

8. Kit-of-parts as claimed in any of claims 1 to 7, in which the grippers of the forceps are biased open to a spaced apart disposition, preferably spaced apart by about 0.1 to 0.3 mm.

9. Kit-of-parts as claimed in any of claims 1 to 8, **characterized in that** the means (20) for intraocularly holding the excised retinal tissue and/or the distance holding means (30) is comprised of a shape-memory alloy, preferably comprising nickel and titanium.

10. Kit-of-parts as claimed in any of claims 1 to 9, wherein either one (24) of the two grippers is stationary and the other (Fig. 4b: 22) of the two grippers is movable upon actuation by movement relative to the sheath (26), or both (Fig. 4a: 22, 22) of the two grippers are movable upon actuation by movement relative to the sheath (26).

11. Kit-of-parts as claimed in any of claims 1 to 10, wherein each lumen of the endoscope has a longitudinal axis, and the grippers and/or the distance holding means laterally project from the longitudinal axis of the respective lumen.

12. Kit-of-parts as claimed in claim 11, wherein the lateral projection from the longitudinal axis of the respective lumen is by between about 0.5 and 3 mm, preferably by between about 1 and 2 mm.

## Patentansprüche

1. Teilesatz zum Behandeln von neovaskulärer Makuladegeneration und genauer Choroidea-Neovaskularisation, wobei der Satz umfasst:
- eine Einrichtung (10, 12) zum intraokularen Herausschneiden von Retinalgewebe,
- eine Einrichtung (20) zum intraokularen Halten des herausgeschnittenen Retinalgewebes, und
- eine Einrichtung (10, 12) zum intraokularen Abtragen des herausgeschnittenen Retinalgewebes, wobei
- die Einrichtung zum intraokularen Abtragen des herausgeschnittenen Retinalgewebes Energie führen und emittieren kann, vorzugsweise Laserlicht, und bevorzugter Laserlicht mit einer Wellenlänge in dem Bereich von 180 bis 400 nm, ferner umfassend
- eine Einrichtung (30) zum Halten des Abstands zwischen der Einrichtung zum intraokularen Halten des herausgeschnittenen Retinalgewebes und der Einrichtung zum intraokularen Abtragen des herausgeschnittenen Retinalgewebes, wobei die Form von zumindest einem distalen Abschnitt der Einrichtung zum intraokularen Abtragen des herausgeschnittenen Retinalgewebes und die Form (32) des distalen Endes der Einrichtung zum Halten des Abstands (30) so angepasst sind, dass sie einander entsprechen, wobei das distale Ende der Einrichtung zum Halten des Abstands einen konkaven Rundungshalbmesser (32) umfasst, vorzugsweise von zwischen ungefähr 1 und 2 mm, wobei die
- Einrichtung zum intraokularen Abtragen des herausgeschnittenen Retinalgewebes zumindest eine Lichtleitfaser (12) umfasst, die in einem Prisma (16) endet, zum Ablenken des Laserlichts, und wobei die
- Einrichtung zum intraokularen Halten des herausgeschnittenen Retinalgewebes eine endoskopische Zange (20) mit zwei Greifern (22, 22; 22, 24) umfasst, und ferner umfassend
- ein Endoskop mit einer Ummantelung (26) und zumindest einem Lumen innerhalb der Ummantelung, wobei die Einrichtung (20) zum intraokularen Halten des herausgeschnittenen Retinalgewebes durch ein Lumen des Endoskops durchgeht, und die Einrichtung zum Halten des Abstands (30) durch ein Lumen des Endoskops durchgeht, wobei die Abstandshalteeinrichtung (30) bei Betätigung durch eine Bewegung relativ zu der Ummantelung (26) bewegbar ist,
- ferner umfassend eine Einrichtung zum Erzeugen von Laserlicht, wobei die Einrichtung zum Erzeugen von Laserlicht betriebsfähig ist, um Laserlicht mit einer Wellenlänge in dem Bereich von 180 bis 400 nm zu erzeugen, vorzugsweise eine Wellenlänge von 193 nm und/oder 308 nm, wobei die Einrichtung zum Erzeugen von Laserlicht ein Excimer-Laser ist.

2. Teilesatz nach Anspruch 1, **dadurch gekennzeichnet, dass** die zumindest eine Lichtleitfaser (12) einen Brechungsindex von ungefähr 1,2 bis 1,5 aufweist, und vorzugsweise eine Quarzfaser oder ein hohler Lichtleiter ist.

3. Teilesatz nach Anspruch 1 oder 2, **dadurch gekennzeichnet, dass** der Durchmesser der zumindest einen Lichtleitfaser (12) von 50 bis 2000 µm ist, vorzugsweise von 100 bis 800 µm, bevorzugter von ungefähr 200 bis 600 µm.

4. Teilesatz nach einem der Ansprüche 1 bis 3, **dadurch gekennzeichnet, dass** die zumindest eine Lichtleitfaser (12) eine distale Lichtausgangsoberfläche (10; 18) mit einem Oberflächenbereich in dem Bereich von 0,002 bis 3 mm² aufweist, vorzugsweise 0,008 bis 0,3 mm², bevorzugter ungefähr 0,03 mm².

5. Teilesatz nach einem der Ansprüche 1 bis 4, **dadurch gekennzeichnet, dass** die Einrichtung zum intraokularen Abtragen des herausgeschnittenen Retinalgewebes zumindest eine Lichtleitfaser umfasst, die an ihrem distalen Ende eine Kappe (14) mit einem Lichtausgang (18), vorzugsweise einem seitlichen Lichtausgang, angebracht hat.

6. Teilesatz nach einem der Ansprüche 1 bis 5, **dadurch gekennzeichnet, dass** die Einrichtung zum intraokularen Abtragen des herausgeschnittenen Retinalgewebes eine Längsachse aufweist, und in die Richtung der Längsachse um zwischen ungefähr 0,5 und 4 mm verschoben werden kann, und/oder um die Längsachse um ungefähr 5° gedreht werden kann, vorzugsweise um ungefähr 10°, bevorzugter um ungefähr 15°, noch bevorzugter um ungefähr 120°.

7. Teilesatz nach einem der Ansprüche 1 bis 6, wobei die Einrichtung zum Erzeugen von Laserlicht betriebsfähig ist, um gepulstes Laserlicht zu erzeugen, wobei die Impulse eine Dauer von zwischen ungefähr 10 ns bis 300 ns aufweisen, und/oder eine Energie in dem Bereich von ungefähr 3 bis 300 mJ/mm².

8. Teilesatz nach einem der Ansprüche 1 bis 7, bei dem die Greifer der Zange in eine beabstandete Anordnung offen vorgespannt sind, vorzugsweise um ungefähr 0,1 bis 0,3 mm beabstandet.

9. Teilesatz nach einem der Ansprüche 1 bis 8, **dadurch gekennzeichnet, dass** die Einrichtung (20) zum intraokularen Halten des herausgeschnittenen Retinalgewebes und/oder die Abstandshalteeinrichtung (30) eine Formgedächtnislegierung beinhaltet, die vorzugsweise Nickel und Titan umfasst.

10. Teilesatz nach einem der Ansprüche 1 bis 9, wobei irgendeiner (24) der zwei Greifer stationär ist und der andere (Fig. 4b: 22) der zwei Greifer bei Betätigung durch eine Bewegung relativ zu der Ummantelung (26) bewegbar ist, oder beide (Fig. 4a: 22, 22) der zwei Greifer bei Betätigung durch eine Bewegung relativ zu der Ummantelung (26) bewegbar sind.

11. Teilesatz nach einem der Ansprüche 1 bis 10, wobei jedes Lumen des Endoskops eine Längsachse aufweist, und die Greifer und/oder die Abstandshalteeinrichtung seitlich von der Längsachse des jeweiligen Lumens vorstehen.

12. Teilesatz nach Anspruch 11, wobei der seitliche Vorsprung von der Längsachse des jeweiligen Lumens bei zwischen ungefähr 0,5 und 3 mm liegt, vorzugsweise bei zwischen ungefähr 1 und 2 mm.

## Revendications

1. Ensemble d'éléments pour traiter la dégénérescence maculaire néovasculaire et plus particulièrement la néovascularisation choroïdienne, l'ensemble comprenant :
- un moyen (10, 12) pour exciser intra-oculairement le tissu rétinien,
- un moyen (20) pour maintenir intra-oculairement le tissu rétinien excisé, et
- un moyen (10, 12) pour enlever intra-oculairement le tissu rétinien excisé, dans lequel
- le moyen pour enlever intra-oculairement le tissu rétinien excisé peut guider et émettre de l'énergie, de préférence une lumière laser, et plus préférentiellement une lumière laser ayant une longueur d'onde dans la plage de 180 à 400 nm, comprenant en outre
- un moyen (30) pour maintenir la distance entre le moyen pour maintenir intra-oculairement le tissu rétinien excisé et le moyen pour enlever intra-oculairement le tissu rétinien excisé, dans lequel la forme d'au moins une partie distale du moyen pour enlever intra-oculairement le tissu excisé et la forme (32) de l'extrémité distale du moyen pour maintenir la distance (30) sont conçues afin de se conformer l'une à l'autre, dans lequel l'extrémité distale du moyen pour maintenir la distance comprend un rayon de courbure concave (32), de préférence entre environ 1 et 2 mm, dans lequel
- le moyen pour enlever intra-oculairement le tissu rétinien excisé comprend au moins une fibre optique (12) se terminant en un prisme (16) pour dévier la lumière laser, et dans lequel
- le moyen pour maintenir intra-oculairement le tissu rétinien excisé comprend une pince endoscopique (20) ayant deux dispositifs de préhension (22, 22 ; 22, 24), et comprenant en outre
- un endoscope ayant une gaine (26), et au moins un passage à l'intérieur de la gaine, dans lequel le moyen (20) pour maintenir intra-oculairement le tissu rétinien excisé passe à travers un passage de l'endoscope, et le moyen pour maintenir la distance (30) passe à travers un passage de l'endoscope, dans lequel le moyen de maintien de distance (30) est mobile lors de la mise en action par un mouvement par rapport à la gaine (26),
- comprenant en outre un moyen pour générer une lumière laser, le moyen pour générer une lumière laser pouvant être mis en oeuvre pour générer une lumière laser ayant une longueur d'onde dans la plage de 180 à 400 nm, de préférence une longueur d'onde de 193 nm et/ou 308 nm, dans lequel le moyen pour générer une lumière laser est un laser Excimère.

2. Ensemble d'éléments selon la revendication 1, **caractérisé en ce que** l'au moins une fibre optique (12) a un indice de réfraction d'environ 1,2 à 1,5, et est de préférence une fibre de quartz, ou un guide de lumière creux.

3. Ensemble d'éléments selon la revendication 1 ou 2, **caractérisé en ce que** le diamètre de l'au moins une fibre optique (12) est de 50 à 2 000 µm, de préférence de 100 à 800 µm, plus préférentiellement d'environ 200 à 600 µm.

4. Ensemble d'éléments selon l'une quelconque des revendications 1 à 3, **caractérisé en ce que** l'au moins une fibre optique (12) a une surface de sortie de lumière distale (10 ; 18) avec une superficie dans la plage de 0,002 à 3 mm², de préférence 0,008 à 0,3 mm², plus préférentiellement environ 0,03 mm².

5. Ensemble d'éléments selon l'une quelconque des revendications 1 à 4, **caractérisé en ce que** le moyen pour enlever intra-oculairement le tissu rétinien excisé comprend au moins une fibre optique ayant un capuchon monté à son extrémité distale (14) avec une sortie de lumière (18), de préférence une sortie de lumière latérale.

6. Ensemble d'éléments selon l'une quelconque des revendications 1 à 5, **caractérisé en ce que** le moyen pour enlever intra-oculairement le tissu rétinien excisé a un axe longitudinal, et peut être déplacé dans la direction de l'axe longitudinal entre environ 0,5 et 4 mm, et/ou peut être tourné autour de l'axe longitudinal d'environ 5°, de préférence d'environ 10°, plus préférentiellement d'environ 15°, toujours plus préférentiellement d'environ 120°.

7. Ensemble d'éléments selon l'une quelconque des revendications 1 à 6, dans lequel le moyen pour générer une lumière laser peut être mis en oeuvre pour générer une lumière laser pulsée, les impulsions ayant une durée entre environ 10 ns et 300 ns, et/ou une énergie dans la plage d'environ 3 à 300 mJ/mm².

8. Ensemble d'éléments selon l'une quelconque des revendications 1 à 7, dans lequel les dispositifs de préhension de la pince sont sollicités dans une position ouverte à une disposition écartée, de préférence écartée d'environ 0,1 à 0,3 mm.

9. Ensemble d'éléments selon l'une quelconque des revendications 1 à 8, **caractérisé en ce que** le moyen (20) pour maintenir intra-oculairement le tissu rétinien excisé et/ou le moyen de maintien de distance (30) comprend un alliage à mémoire de forme, comprenant de préférence du nickel et du titane.

10. Ensemble d'éléments selon l'une quelconque des revendications 1 à 9, dans lequel l'un ou l'autre (24) des deux dispositifs de préhension est immobile et l'autre (figure 4b : 22) des deux dispositifs de préhension est mobile lors de la mise en action par un mouvement par rapport à la gaine (26), ou les deux (figure 4a : 22, 22) des deux dispositifs de préhension sont mobiles lors de la mise en action par un mouvement par rapport à la gaine (26).

11. Ensemble d'éléments selon l'une quelconque des revendications 1 à 10, dans lequel chaque passage de l'endoscope a un axe longitudinal, et les dispositifs de préhension et/ou le moyen de maintien de distance dépassent latéralement de l'axe longitudinal du passage respectif.

12. Ensemble d'éléments selon la revendication 11, dans lequel la partie en saillie latérale de l'axe longitudinal du passage respectif fait entre environ 0,5 et 3 mm, de préférence entre environ 1 et 2 mm.
